# EUROPEAN PATENT SPECIFICATION

(11) **EP 2 854 583 B1**
(45) Date of publication and mention of the grant of the patent: **16.11.2016**
(21) Application number: 13729050.8
(22) Date of filing: 28.05.2013
(51) Int. Cl.: A42B 1/06

(54) **HEADGEAR TO REDUCE TRAVEL SICKNESS**
KOPFBEDECKUNG ZUR MINDERUNG VON REISEKRANKHEIT
COUVRE-CHEF POUR RÉDUIRE LE MAL DES TRANSPORTS

(30) Priority: 25.05.2012 GB 201209320
(43) Date of publication of application: 08.04.2015
(73) Proprietor: Flaxman, Timothy, Great Yarmouth, Norfolk NR31 9AJ (GB)
(72) Inventor: Flaxman, Timothy, Great Yarmouth, Norfolk NR31 9AJ (GB)
(74) Representative: Cummings, Sean Patrick
(86) International application number: PCT/GB2013/051411
(87) International publication number: WO 2013/175245

(56) References cited:
- EP-A1- 0 729 715
- WO-A1-2008/122788
- GB-A- 331 354
- US-A1- 2006 242 751
- US-A1- 2010 186 145
- US-B1- 6 275 998

## Description

### Field of the Invention

The present invention relates to the technical field of a device to reduce or prevent feelings of travel sickness in a user. In particular headgear such as a cap is provided for the user to wear during a journey.

### Background to the Invention

Travel sickness or kinetosis is a common condition which is typically caused by repeated lateral movements whilst travelling. So for example, a person afflicted with this condition may not suffer the symptoms- nausea or vomiting - whilst travelling in a straight line but may suffer when the direction of travel changes at intervals, especially irregular or sudden intervals. The mode of transport causing the symptoms will vary between sufferers, but can be a car or a coach, a boat, aeroplane or by going on certain fairground rides such as a rollercoaster or a merry-go-round, in severe cases symptoms can persist for several hours following completion of a journey. In some cases travel sickness can be brought on through watching moving images, particularly where the images show rapidly changing motion, on a television or at the cinema.

A number of remedies has been proposed to alleviate the symptoms or to remove them entirely. Medications are available either over the counter or by prescription. For example, antihistamines such as Promethazine are used but can cause significant drowsiness as a side effect. Also, alkaloids such as scopolamine are known to be used in the form of transdermal patches, but extreme care must be taken due to the side effects. Non-chemical methods are also known which can be as simple as sleeping throughout the journey or keeping in the fresh air. Alternatively, sufferers from car or coach sickness are advised simply to look through the front windscreen of the vehicle and to refrain from looking out through the side windows.

WO2008/122788 discloses a pair of spectacles to reduce travel sickness in a user.

It is an object of the present invention to provide a cap which will substantially reduce or prevent the wearer from suffering the effects due to travel sickness.

### Summary of the Invention

According to a first aspect of the invention there is provided headgear comprising one or more flaps the or each flap being attached to the headgear and deployable over an eye of a wearer, such that one eye remains uncovered, and characterised in that the or each flap is sufficiently opaque to light to prevent the wearer from perceiving images through said flap when said flap is deployed.

Preferably, the or each flap is removably attachable, to enable the headgear to be used as normal when a user is not travelling.

Advantageously, the duration of eye coverage by a flap is determined by the use of an integral electrical timer to give visual or audible indication of time elapsed. Further advantageously said timer is coupled to an electromechanical device to deploy or retract a flap. Optionally, the opacity of a flap is complete, but allows light to enter the eye around the periphery of the flap.

Conveniently individual eye coverage is achieved by means of a vertically or horizontally sliding flap.

Preferably individual eye coverage is achieved by means of one flap hinged in such a way that either eye can be covered by the flap.

The headgear is preferably a peaked cap, the or each flap being attached beneath the peak of the cap so that a flap is close to the eye region. A flap can advantageously be raised and fastened to the underside of the peak such that the flap is minimally visible to onlookers to allow the cap to function as a normal cap.

According to a second aspect of the invention there is provided a flap, suitable for attachment to headgear, including a cap, which can be temporarily or permanently fixed to said headgear or cap.

### Brief Description of the Drawings

The invention will now be described with reference to the accompanying drawings which show by way of example only three embodiment of headwear. In the drawings:
Figure 1 is a perspective view of a cap in accordance with a first embodiment of the invention.
Figure 2 is a side view of a cap in accordance with a first embodiment of the invention.
Figure 3 is a perspective view of a sun visor in accordance with a second embodiment of the invention.
Figure 4 is a perspective view of a head band in accordance with a third embodiment of the invention.
Figure 5(a) and 5(b) is a schematic side elevation of a cap in accordance with a fourth embodiment of the invention.
Figure 6 is a perspective view of attachable flaps in accordance with a fifth embodiment of the invention.

### Detailed Description of the Invention

In its basics, the invention provides head wear in the form of a cap or hat, or a sun visor or a simple head band to be worn by a person who suffers from travel sickness, from which an attached flap or flaps hanging downwards from the head wear, restrict the view of one eye to at most a peripheral image from around the edge of the flap. Normally only one flap is used at a time, but by providing two flaps the view of each eye in turn can be restricted for an equal duration thereby mitigating fatigue.

Without being bound to theory it is believed that sickness arises from a mismatch of the three-dimensional image formed in the brain, together with sensory impulses received from the balance mechanism within the ear. By removing the ability of the user to perceive a three-dimensional image in preventing the perception of movement in one eye, but still allowing observation of the user's surroundings in the other eye, the mismatch is tampered with or removed and the travel sickness resulting therefrom does not occur. Critically, the eye which has vision restricted should remain open and should receive some illumination. If the eye which has vision restricted is shut or has no illumination then the brain seemingly disregards any signal coming from it and travel sickness ensues. It is envisaged that in order to be most effective, the invention should be worn immediately prior to the journey and for the entire duration of the journey.

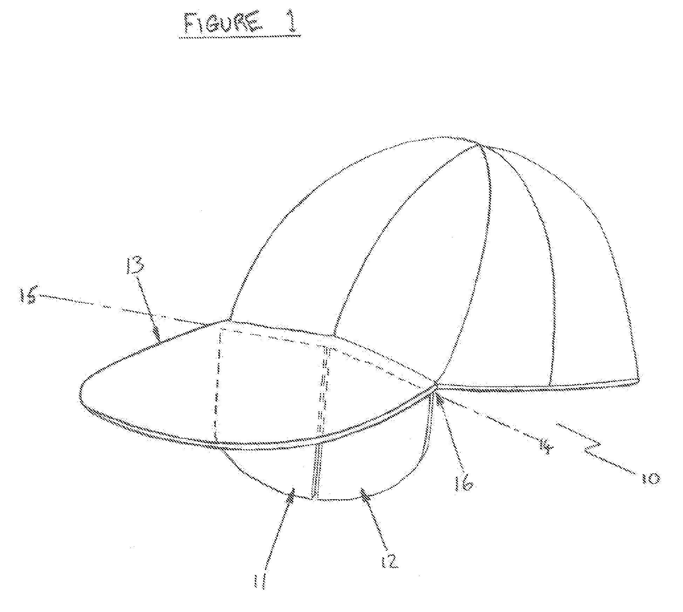

In Figure 1, a baseball type cap, generally referenced 10, is shown. Two flaps 11 and 12 made from fabric or other material, are shown hanging downwards from the cap peak 13, and are individually hinged at axis lines 14 and 15. The flaps 11, 12 are separately moveable between a vision restricting position and a stowed position where they are removed from restricting the visual field.

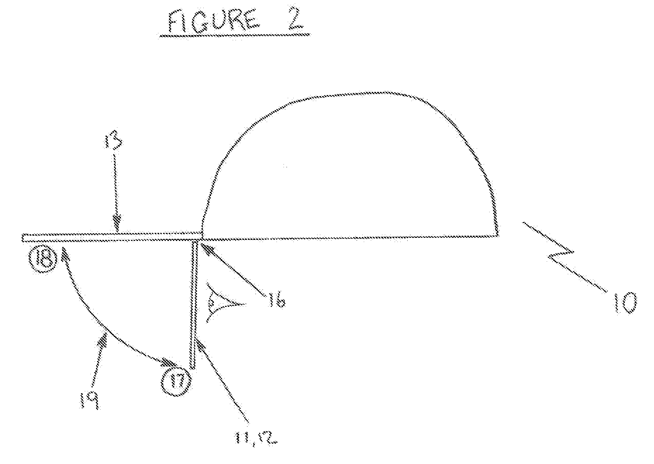

In Figure 2, the baseball type cap 10 is shown in side elevation to illustrate mechanical operation of the invention and the movement of flaps 11, 12 between the two positions. The arc 19, shows the limit of travel of the flaps 11, 12 from position 17 where flap 11, 12 restricts the visual field of an eye, to position 18 where the flap does not.

The flaps 11 and 12, are hinged starting at a point 16 and along the line 14, 15, enabling movement of the flaps 11, 12 to a position directly underneath and adjacent to the peak 13, to position 18, whereby they can be temporarily fastened and at which stage they no longer restrict vision.

The flaps 11, 12 are opaque to visible light to such an extent that the user cannot perceive an image through the flaps 11 and 12. Complete blockage of light by a flap 11, 12 is normally undesirable. It is therefore understood that although a flap can be fully opaque there is no necessity for the flap to be completely so: only that insufficient light passes through to allow the eye and brain to discern individual objects and to perceive motion. When wearing the above cap 10 therefore, the user's uncovered eye, being unrestricted by the flap 11 or 12 which is fastened underneath the cap peak 13, receives a normal image therethrough but the eye behind the opaque flap only receives light from around the periphery of the cap 10. The user is thereby rendered less susceptible to travel sickness. The flaps 11 and 12 can be secured to the cap peak 13 at position 18 by a number of means. Firstly a Velcro™ fixing would allow simple operation by the user to facilitate coverage of each eye throughout a journey to be of equal duration. If the cap has to be removed during a journey to enable alternating eye coverage then travel sickness can occur quickly. In addition, fastening of the flaps 11 and 12 to the cap peak at position 18 can be achieved by pop-studs or buttons or magnetism.

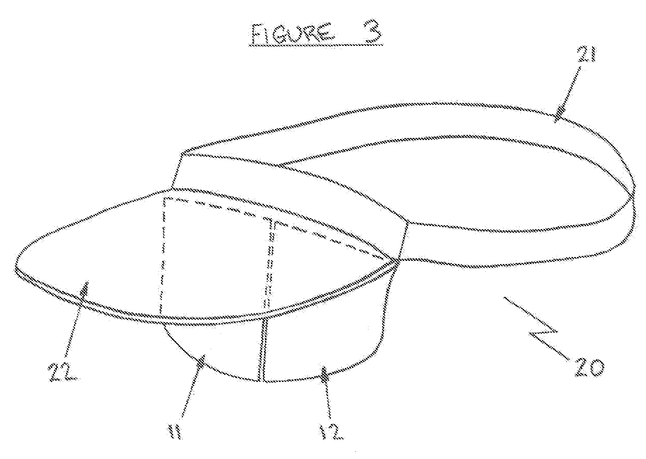

In Figure 3, a sun visor, generally referenced 20, is shown as a further embodiment of the invention. In this example, a head band 21, is used to support the flaps 11 and 12 underneath the sun visor 22.

A benefit of the invention is the facility to fasten both flaps underneath the peak at the same time allowing the user to employ the head wear for normal use. Moreover a further benefit of the invention is the ability to allow both eyes to be covered at the same time to aid sleep or relaxation, although such sleeping caps are known per se, see for example US2010/0186145. In addition, decorations can be added to the flaps to, for example, increase the appeal of the head wear to children, a group which is particularly prone to travel sickness.

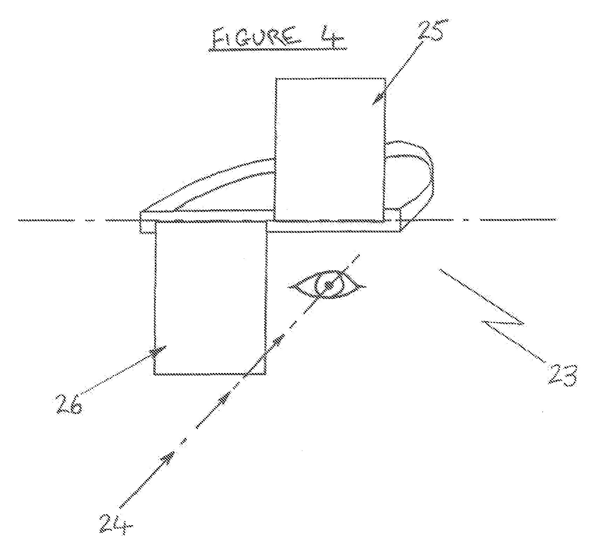

In Figure 4, a head band, generally referenced 23, is shown demonstrating in this example, the line of sight 24 in the left eye to be clear of restriction by the flap 25 and the right eye covered by flap 26. In this embodiment, the flaps 25 and 26 can each slide vertically within slots (not illustrated) in the headband 23.

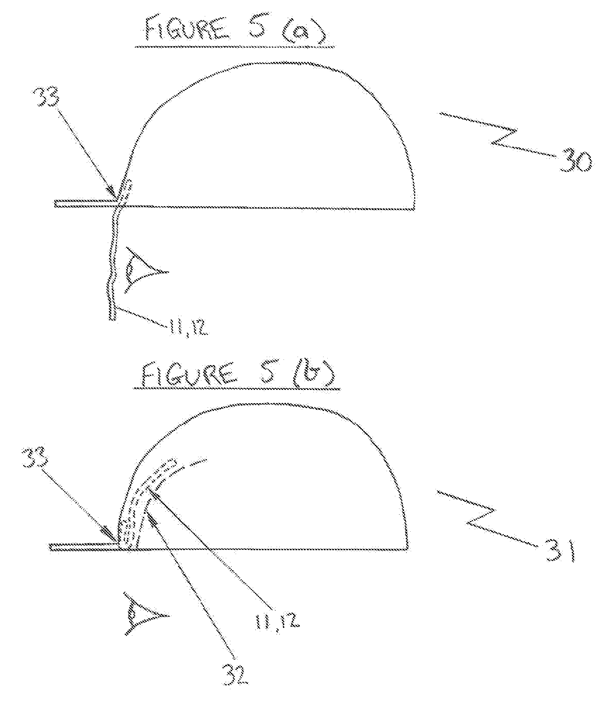

A further embodiment of the invention in Figure 5(a), shows a cap generally referenced 30 having optionally removable flaps 11,12, made of fabric or other material, temporarily attached to the cap at point 33, hanging downwards and restricting vision. In Figure 5(b), a cap, generally referenced 31 has the flaps 11, 12 optionally tucked inside the cap 31 resting on the user's forehead 32.

Optionally the flaps are removably attached to the cap enabling the cap to be used as normal when the user is not travelling. Moreover in this embodiment the flaps can be attached to any cap or head wear using temporary fastening methods such as Velcro™ or pop-studs or buttons or other temporary fastening methods.

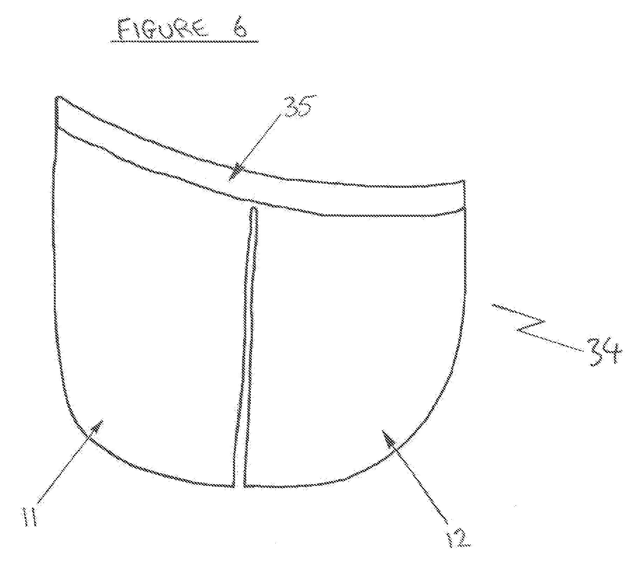

In Figure 6, generally referenced 34, optionally attachable flaps 11, 12 are shown. The area shaded 35, can be optionally temporarily fastened inside the front rim of any suitable hat or head wear using Velcro™ or other suitable temporary fixing method.

In an additional embodiment a pre-set duration of eye coverage can be achieved by the use of an integral electrical timer to give visual or audible indication of time elapsed with or without a coupled electromechanical device to deploy the flaps. In a further additional embodiment one or more areas of a flap, for example the in-use lower edge can include an adhesive material or material which clings to a user's skin, to ensure that the flap remains close to the user's face and is not easily moved, for example by air-current. Other means of reducing the movement of a flap when in-use, well known in the art, can also be included.

It will of course be understood that the invention is not limited to the specific details described herein, which are given by way of example only, and that various modifications and alterations are possible within the scope of the invention, as defined by the claims.

## Claims

1. Headgear (10) comprising one or more flaps (11, 12), the or each flap (11, 12) being attached to the headgear (10) and deployable over an eye of a wearer, such that one eye remains uncovered, and **characterised in that** the or each flap (11, 12) is sufficiently opaque to light to prevent the wearer from perceiving images through said flap (11, 12) when said flap (11, 12) is deployed.

2. Headgear according to Claim 1 wherein the or each flap is removably attachable.

3. Headgear according to Claim 1 or Claim 2 wherein the duration of eye coverage by a flap is determined by the use of an integral electrical timer to give visual or audible indication of time elapsed.

4. Headgear according to claim 3, wherein said timer is coupled to an electromechanical device to deploy or retract a flap.

5. Headgear according to any preceding claim, wherein a flap is opaque and so shaped to allow light to enter the eye around the periphery of said flap when deployed.

6. Headgear according to any preceding Claim wherein individual eye coverage is achieved by means of a vertically or horizontally sliding flap.

7. Headgear according to any preceding Claim wherein individual eye coverage is achieved by means of one flap hinged in such a way that either eye can be covered by the flap.

8. Headgear according to any preceding Claim wherein the headgear is a peaked cap, the or each flap being attached beneath the peak (13) of the cap.

9. Headgear according to claim 8 wherein a flap can be raised and fastened to the underside of the peak such that the flap is minimally visible to onlookers.

## Patentansprüche

1. Kopfbedeckung (10), die eine oder mehrere Klappen (11,12), wobei die oder jede Klappe (11, 12) an der Kopfbedeckung angebracht ist (10) und entfaltbare über einem Auge eines Trägers, so daß ein Auge unbedeckt bleibt, und **dadurch gekennzeichnet, dass** die oder jede Klappe (11,12), um Licht ausreichend opak ist, den Träger vor der Wahrnehmung Bilder durch die Klappe (11,12), wenn die Klappe (11,12) eingesetzt zu verhindern.

2. Kopfbedeckung nach Anspruch 1, wobei die oder jede Klappe lösbar befestigbar ist.

3. Kopfbedeckung nach Anspruch 1 oder Anspruch 2, wobei die Dauer der Augenabdeckung durch eine Klappe durch die Verwendung eines integralen elektrischen Zeitgeber bestimmt wird, optische oder akustische Anzeige der verstrichenen Zeit zu geben.

4. Kopfbedeckung nach Anspruch 3, wobei der Zeitgeber auf eine elektromechanische Vorrichtung gekoppelt ist, bereitstellen oder eine Klappe zurückzuziehen.

5. Kopfbedeckung nach einem der vorhergehenden Ansprüche, wobei eine Klappe opak ist und so geformt ist, damit Licht, das Auge um den Umfang der Klappe einzugeben, wenn im Einsatz.

6. Kopfbedeckung nach einem der vorhergehenden Ansprüche, wobei einzelne Augenabdeckung mittels eines vertikal oder horizontal gleitende Klappe erreicht wird.

7. Kopfbedeckung nach einem der vorhergehenden Ansprüche, wobei einzelne Augenabdeckung mittels einer Klappe derart angelenkt ist erreicht, daß entweder Auge kann durch die Klappe abgedeckt werden.

8. Kopfbedeckung nach einem der vorhergehenden Ansprüche, wobei der Kopfbedeckung ein Schirmmütze ist, wobei die oder jede Klappe wobei unterhalb der Spitze (13) der Kappe angebracht ist.

9. Kopfbedeckung nach Anspruch 8, wobei eine Klappe kann so an der Unterseite des Spitzen angehoben und befestigt werden, dass die Klappe an die Zuschauer minimal sichtbar ist.

## Revendications

1. Coiffe (10) comprenant un ou plusieurs volets (11,12), le ou chaque volet (11, 12) étant fixé à la coiffe (10) et déployable sur un oeil d'un porteur, de telle sorte que l'oeil reste à découvert, et **caractérisé en ce que** le ou chaque volet (11,12) est suffisamment opaque à la lumière pour empêcher l'utilisateur de percevoir des images à travers ledit volet (11,12) lorsque ledit volet (11, 12) est déployé.

2. Coiffe selon la revendication 1, dans lequel le ou chaque volet est fixé de manière amovible.

3. Coiffe selon la revendication 1 ou la revendication 2, dans lequel la durée de la protection oculaire par un clapet est déterminée par l'utilisation d'une minuterie électrique intégré pour donner une indication visuelle ou audible du temps écoulé.

4. Coiffe selon la revendication 3, dans lequel ledit temporisateur est couplé à un dispositif électromécanique pour déployer ou rétracter un rabat.

5. Coiffe selon l'une quelconque des revendications précédentes, dans lequel un volet est opaque et donc en forme pour permettre à la lumière d'entrer dans l'oeil autour de la périphérie dudit volet lorsqu'il est déployé.

6. Coiffe selon l'une quelconque des revendications précédentes, dans lequel la couverture de l'oeil individuel est réalisé au moyen d'un volet coulissant verticalement ou horizontalement.

7. Coiffe selon l'une quelconque des revendications précédentes, dans lequel la couverture de l'oeil individuel est réalisé au moyen d'un rabat à charnière d'une manière telle que les deux yeux peut être couvert par le rabat.

8. Coiffe selon l'une quelconque des revendications précédentes, dans lequel le couvre-chef est une casquette, le ou chaque volet étant fixé sous le pic (13) de la capsule.

9. Coiffe selon la revendication 8, dans lequel un rabat peut être relevé et fixé sur la face inférieure de la pointe de telle sorte que le rabat est peu visible aux spectateurs.
